# EUROPEAN PATENT SPECIFICATION

(11) **EP 3 878 480 B1**
(45) Date of publication and mention of the grant of the patent: **07.06.2023**
(21) Application number: 21161635.4
(22) Date of filing: 09.03.2021
(51) Int. Cl.: A61L 9/20, F24H 3/04, H05B 3/00

(54) **ELECTRIC CONVECTOR WITH BACTERICIDAL EFFECT**
ELEKTRISCHER KONVEKTOR MIT BAKTERIZIDER WIRKUNG
CONVECTEUR ÉLECTRIQUE À EFFET BACTÉRICIDE

(30) Priority: 11.03.2020 EP 20472002
(43) Date of publication of application: 15.09.2021
(73) Proprietor: Tesy OOD, 9701 Shumen (BG)
(72) Inventor: KYURKCHIEV, Zhechko Angelov, 1166 Sofia (BG); IVANOV, Plamen Ivanov, 1166 Sofia (BG)
(74) Representative: Benatov, Samuil Gabriel

(56) References cited:
- CN-A- 107 812 227
- CN-U- 205 683 324
- RO-A2- 132 030
- RO-B1- 112 398
- US-A1- 2013 291 735
- Sameen Ahmed Malik ET AL: "Comparison of standard light-emitting diode (LED) and 385 nm ultraviolet A LED (UVA-LED) for disinfection of Escherichia coli", Malaysian Journal of Fundamental and Applied Sciences, 17 December 2017 (2017-12-17), pages 430-437, XP055723004, DOI: 10.11113/mjfas.v13n4-2.758 Retrieved from the Internet: URL:https://www.researchgate.net/publicati on/321903841_Comparison_of_standard_light- emitting_diode_LED_and_385_nm_ultraviolet_ A_LED_UVA-LED_for_disinfection_of_Escheric hia_coli/fulltext/5b1ec2b8aca272277fa7009a /Comparison-of-standard-light-emitting-dio de-LED-and-385-nm-ultraviolet-A-LED-UVA-LE D-for-disi [retrieved on 2021-07-26]

## Description

### TECHNICAL FIELD OF THE INVENTION

The electric convector with bactericidal effect is applicable for heating closed premises and at the same time disinfecting the air, and it is suitable for both household and industrial purposes.

### PRIOR ART

It is well known that UV radiation affects organisms, and particularly microorganisms, in a negative way. Short wavelength UV radiation (UVC) causes damage to the microorganisms' DNA thus making them unable to reproduce. This fact has been used for the creation of many devices for air disinfection in closed premises. There are disinfecting or sterilizing devices that use artificially induced airflow, for instance created by fans or air pumps (EP2455678A1, WO2019106689A1).

Air-conditioning and heating systems that use UV radiation for air disinfection are known (CN107812227A, WO20 19045778A1, US2013291735, RO112 398).

A device for air sterilization with UV radiation with natural air circulation is known. It consists of a housing with a lower air inlet opening and an upper air outlet opening, in the housing there is installed a source of UV radiation and heating elements in the form of filament lamps for heating the air and for creating vertical convection airflow inside the housing which is irradiated by a source of UV radiation. The device is not intended for heating the room where it is placed and the purpose of the air heating is only to create natural thermal convection of the air in the device.

There exists a device for air disinfection with UV radiation, intended for installation on electric convector (Romanian patent application No. RO132030 A2). The device consists of a housing in which one or two mercury gas-discharge lamps radiating across the whole UV spectrum are installed. The device is to be installed on electric convector with natural circulation above the outlet for the heated air so that the airflow passes around the gas-discharge lamps. A disadvantage of the device is that the mercury gas-discharge lamps radiate across the whole UV spectrum (UV-A; B; C), and particularly (UV-B; C) are potentially harmful for humans and animals; for this reason, the device has a presence sensor which disables the UV lamps when the presence of a person or an animal is detected. Another disadvantage is the ozone generation during operation of the device. And also, another disadvantage is the undesired heating of the UV lamps by the heated airflow.

There exists a heating device with natural convection which purifies air (CN105546622). The device consists of a housing in which there is installed a heating element that creates a natural convection airflow and also a photo-catalytic plate and at least one ultraviolet lamp located on the path of the heated air from the heating element to the air outlet in the housing. The photo-catalytic elements and the ultraviolet lamps kill the microorganisms in the heated airflow that passes around them before it leaves the housing of the heating device. A disadvantage of the device is the undesired heating of the ultraviolet lamps located near the heating element. Another disadvantage is that the UV lamps radiate across the whole UV spectrum, including the ranges that are dangerous for humans. And another disadvantage of the device is that part of the radiated energy in the infrared spectrum is absorbed by the photo-catalytic panel, and the exhaust air passes through the side panels and thus the velocity of circulation along the height of the panel is uneven. This leads to uneven treatment of the air which passes through the device.

The majority of UV lamps generate ozone which has negative effect on human health. The ozone molecule consists of 3 oxygen atoms. It readily decomposes, and instead of one ozone, the result is one ordinary two-atom oxygen molecule and one free oxygen atom. The latter is particularly aggressive and it is what makes ozone one of the strongest oxidizers. Ozone penetrates and manifests its toxic effect through the respiratory system. The health effects are manifested through inflammations of the respiratory organs, reduction in the lung performance accompanied by quickened breathing. It affects the immune system and reduces the resistance to respiratory diseases. The toxic effect of ozone is manifested through oxidation of the sulfhydryl and amino groups of enzymes, co-enzymes, proteins and peptides. It also oxidizes unsaturated fatty acids to fatty peroxides. For this reason, any devises that use UV radiation and generate ozone are dangerous for humans and animals.

### SUMMARY OF THE INVENTION

The aim of the invention is to create an electric convector with natural air convection which at the same time disinfects the air passing through it by UV radiation. The additional aim of the invention is the electric convector to be energy efficient. Another additional aim of the invention is to use such UV radiation that is safe for the users.

These and other aims are fulfilled by creating an electric convector in accordance with the invention, which consists of a housing with at least one air inlet opening located in the lower wall/part of the housing and least one outlet opening for the heated air located in the upper part of the housing, whereby the following components are located in the housing: an electric heating element with heating temperature in the range of 200 °C - 450 °C, an electronic control unit adapted to operate the electric heating element and means for connecting to source of electrical energy. In accordance with the invention, the electric convector further comprises at least one source of UV radiation that is a UV light emitting diode (LED), emitting UV light in the UVA spectral range (315-380 nm) with power of more than 150 mW and is located beneath the electric heating element, between the latter and the at least one air inlet opening, on the path of the airflow.

In one embodiment the UVA source is mounted inside the convector housing between the electric heating element and the inlet opening/s, on the path of the incoming airflow.

A variant is possible in which the UVA source is mounted outside the convector housing, on the path of the incoming airflow. Moreover, the performance of the UVA source can be independent of the operation of the convector (connected to the source of electrical energy, separately from the convector) or by an additional connection to the electronic control unit of the convector.

Preferably, the distance between the UV light emitting diodes (LEDs) and the electric heating element should be not less than 30 mm.

It is possible the electronic control unit to include at least a microprocessor, memory, timer/time measuring device, internet module and wireless connection module.

The number of UV light emitting diodes is determined by the emission angle of the diode, the power of radiation, the location and size of the air inlet openings.

Preferably, the UV light emitting diodes should be distributed evenly above or below the air inlet openings.

An advantage of the electric convector, in accordance with the invention, is that it has enhanced energy efficiency because it uses UV light emitting diodes which emit in the long wavelength UVA spectrum and have maximum efficiency of the emitted light energy, versus the applied electrical energy, and additionally, for its disinfecting action, the convector uses the combined effect of ultraviolet light from the light emitting diodes and infrared light from the heating element. Another advantage is that UV light emitting diodes are relatively cheaper than other sources of UV radiation. And another advantage of the electric convector is that it is safer for users because UV rays across the UVA spectrum are the least harmful ones for humans and animals, and the reflected UV light inside the volume of the housing where the heating element is located is of very low intensity and energy and it has no harmful effect on humans, plants and animals. Apart from that, the energy of the emitted light is in the range of 3.10 - 3.94 eV and it does not generate ozone.

### BRIEF DESCRIPTION OF THE DRAWINGS

The invention is disclosed in details with a preferred embodiment of the electric convector, in accordance with the invention, given as a non-restrictive example, with reference to the attached drawings wherein:
Figure 1 is a view of an embodiment of the convector, in accordance with the invention in cross section.
Figure 2 is a longitudinal section of an embodiment of the convector, in accordance with the invention with 5 light emitting diodes and enlarged partial view.
Figure 3 is a longitudinal section of an embodiment of the convector, in accordance with the invention with 20 light emitting diodes and enlarged partial view.
Figure 4 is a longitudinal section of an embodiment of the convector, in accordance with the invention with groups of low-power light emitting diodes and enlarged partial view.
Figure 5 represents an embodiment of the convector according to the invention in which the UV source is mounted outside the convector housing, on the path of the incoming air flow. A longitudinal section and a cross section of the convector and enlarged partial views of the installation site and the UV source are presented. Also, the separate housing with UV source is shown separately.

### EXEMPLARY DESCRIPTION OF THE INVENTION

In the present application the terms "lower", "upper", "above", "below", "beneath" and "underneath" reflect the usual operational position of the electric convector as shown on the figures.

The electric convector, in accordance with the invention, consists of a housing 1, with inlet 4 and outlet 5 openings for the airflow, an electric heating element 2, means for connecting to source of electrical energy, a precision thermometer 7, a thermal protection 6, and a control unit, located inside the housing 1 and a source of UV radiation, for example UV light emitting diodes 3.

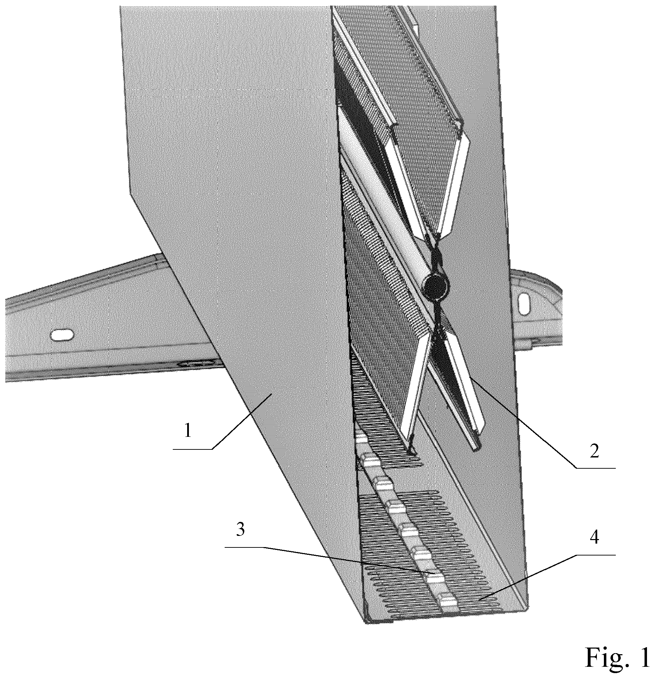

In one embodiment the UV source is mounted inside the housing 1 of the convector (Figs 1-4).

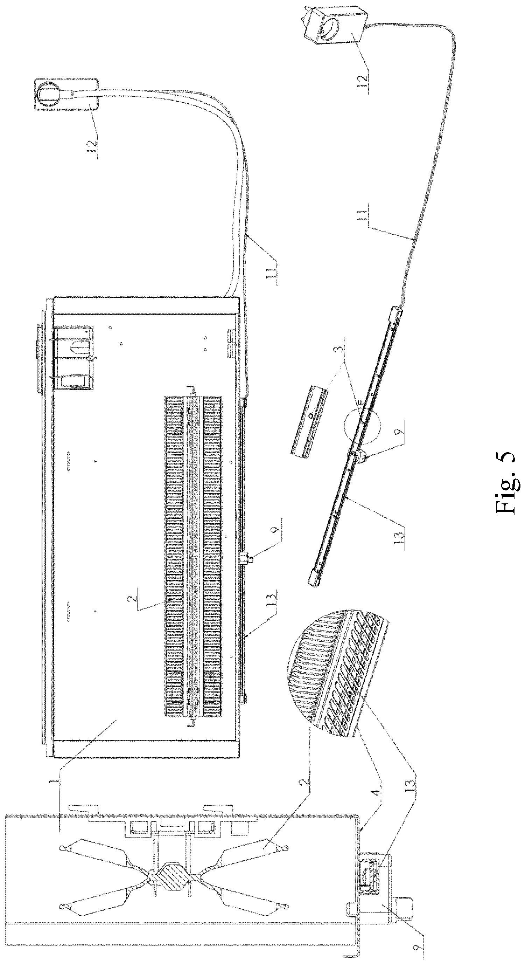

In another embodiment the UV source is mounted outside the convector housing 1 (Fig. 5).

The housing 1 of the electric convertor has to be temperature-resistant and it may be made of metal, glass, or another suitable material. The volume of the housing 1 preferably should be from 0.01 to 0.03 m³ per installed kW of electric power of the heating element 2. In the lower part of the housing there are air inlet openings 4. Usually, these openings 4 are located in the lower wall of the housing 1. The air outlet openings 5 are located in the upper part of the housing 1, for example, in the upper wall of the housing 1 or in the upper part of the front panel of the housing.

The electric heating element 2 operates in the temperature range of 200 °C - 450 °C. Preferably the electric heating element 2 should be located in the lower half of the convector housing 1, thus providing conditions for proper airflow convection. Heating elements with big surface area are preferred, providing maximum and even treatment of the airflow. The heating element 2 may be a heating element of the resistive conductor type. It is possible to use other types of heating elements, for example, but not only, of the printed or thin-layer heating elements type, inductively heated.

The UV light emitting diodes 3 are located beneath the electric heating element 2 on the path of the incoming airflow, preferably at a distance not less than 30 mm from the electric heating element 2, and more preferably - 40 mm, in order not to get heated by the electric heating element. In accordance with the invention the used UV light emitting diodes emit in the UVA spectral range (315-380 nm) with power greater than 150 mW. The energy of the emitted light is preferred to be in the range of 3.10 - 3.94 eV. The service life of UV light emitting diodes with such parameters is more than 50 000 hours which makes them economically very affordable.

The number of UV light emitting diodes 3 depends on the emission angle of the diode, the power of the emitted energy (luminous flux) and the surface of the air inlet opening/openings 4. The emission angle of the light emitting diode depends on the construction of the light emitting diode (mainly the lens, through which the light passes) and shows the manner in which the diode emits in the space towards the central axis of symmetry. The emission angle determines the intensity of radiation at various solid angles. With smaller solid angles the energy is almost entirely focused in the sector outlined by this angle, and at the same time the illuminated area is smaller. In this case the light emitting diodes 3 have to be located closer to one another in order to ensure overlap of the illuminated sections of the heating element 2. With bigger solid emission angle, the energy is distributed within its perimeter, and respectively, the intensity is lower, and the illuminated area is bigger, respectively the number of light emitting diodes 3 may be smaller.

The UV light emitting diodes 3 are distributed evenly above or below the air inlet opening/openings 4 so that they ensure even treatment of the airflow simultaneously by UV and infrared radiation and also ensure maximum surface area of the j oint action of the UV and infrared light.

Preferably, the light emitting diodes 3 may be located in the plane of the air inlet openings 4, thus increasing the distance to the electric heating element 2 and therefore the period of joint action of the UVA radiation from the light emitting diodes 3 and the infrared radiation from the electric heating element 2.

It is possible to use COB diodes (composed of large number of powerful small diodes).

Depending on the diode's emission angle, the intensity of the emitted energy, the position and size of the air inlet openings 4, various embodiments may be implemented which would be able to efficiently reduce the rate of bacteria, viruses and micromycetes in the room.

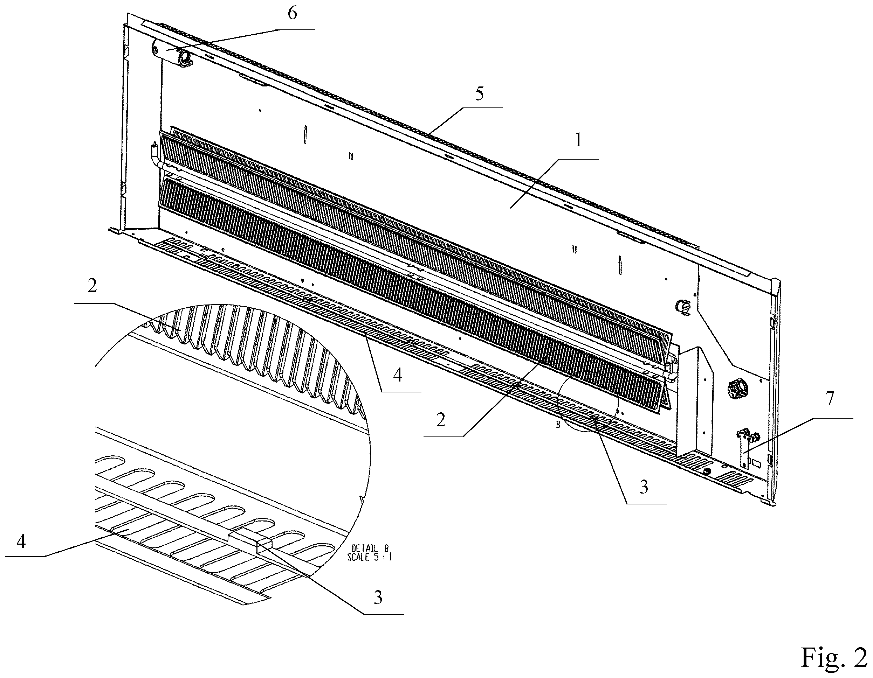

With powerful diodes (more than 1W) and wide emission angle (100-140 degrees), it is possible to use small number of light emitting diodes 3 which fulfil the condition for even illumination of the surface of the electric heating element 2 and luminous intensity carrying sufficient amount of energy, whereby together with the joint action of the radiated infrared light from the electric heating element 2, this will damage important bonds that underlie the stable structure of bacteria, viruses and micromycetes. This embodiment is presented in Figure 2.

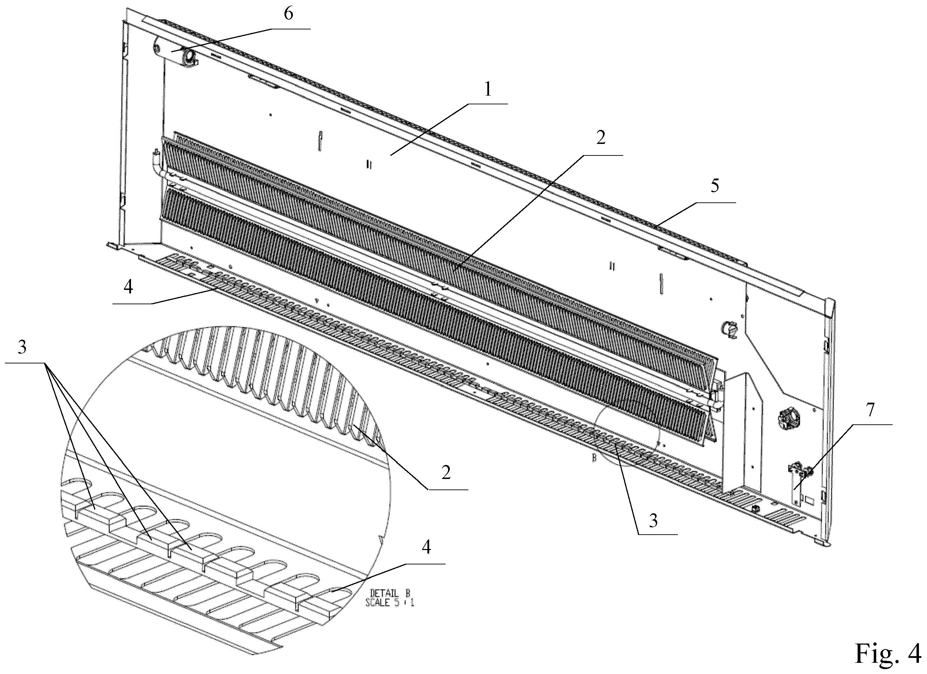

With diodes of medium or low power (0.2 W - 1W) and wide emission angle (100 - 140 degrees), it is possible to use bigger number of light emitting diodes 3 (grouped or evenly distributed) which again fulfil the condition for even illumination of the heating element's surface and luminous intensity carrying sufficient amount of energy, whereby together with the joint action of the radiated infrared light from the electric heating element, this will damage important bonds that underlie the stable structure of bacteria, viruses and micromycetes. An embodiment with grouped diodes is presented in Figure 4.

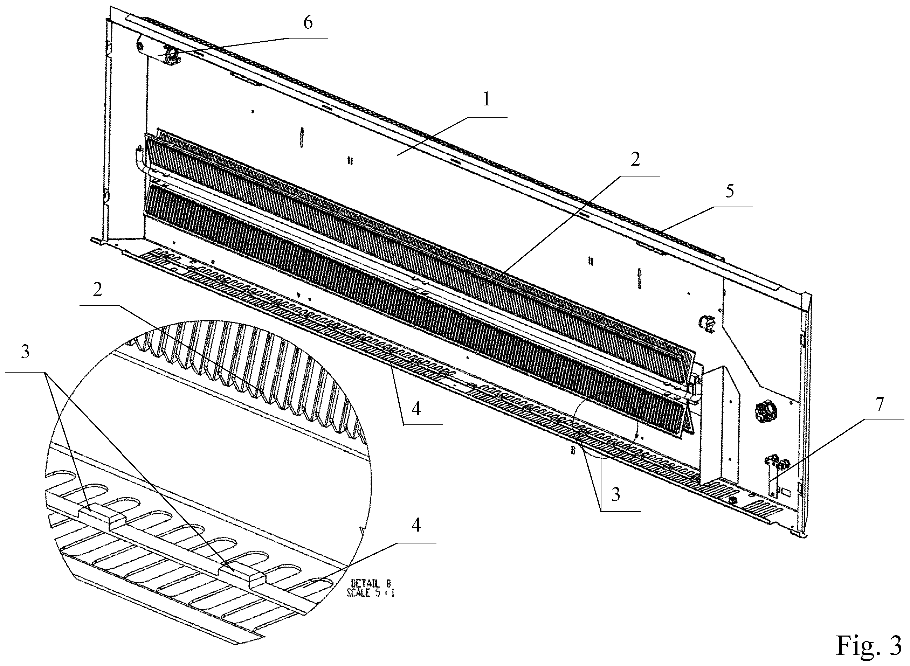

With diodes of high, medium or low power (more than 1W) and narrow emission angle (30-90 degrees), it is possible to implement an embodiment where the electric heating element 2 is positioned vertically (other variants of positioning are also possible) whereby this increases the time for the joint action with the radiated infrared light from the electric heating element 2. With this embodiment, it is possible to reduce the power of the emitted energy by the UVA light emitting diodes 3 or the temperature of the electric heating element 2 whereby the time for joint action of the incoming air is increased. With this embodiment again the condition for sufficient amount of energy for breaking important bonds that underlie the stable structure of bacteria, micromycetes and viruses may be fulfilled. This embodiment is presented in Figure 3.

The control unit of the convector includes a precision thermometer 7 connected to an electronic or mechanical unit that operates the electric heating element 2 based on a set program or on information from the thermometer, power supply unit for the light emitting diodes (LEDs) that is current-operated for accurate implementation of the set mode and safe operation. The convector may have means for local control consisting of a display (or a LED indication) and buttons. The convector may be operated by a remote control through an infrared port, WIFI, Bluetooth or another wireless connection, using a mobile or desktop application installed on a remote device.

### Description of the operation of the invention

Upon switching on the convector, the electronic control unit checks the ambient temperature and, if the conditions allow operation (the set temperature is higher than the ambient temperature in the room), it furnishes voltage to the electric heating element 2 and to the control unit for the LEDs' operation. If the user has allowed (switched on) operational mode of the LEDs module, then the power supply unit of the LEDs furnishes voltage and current in accordance with the LEDs' optimal mode of operation or in accordance with mode of operation set by the user.

The increasing temperature of the electric heating element 2 leads to natural convection of the air in the room, whereby colder air enters the lower openings 4 of the convector and passes by the UVA LEDs 3. Thus, the molecules of gases, bacteria, micromycetes (fungi), etc. obtain energy resulting from the joint action of the emitted energy of the UVA LEDs 3 and the infrared radiation from the electric heating element 2. This impact lasts for the whole duration of the passing of the airflow through the convector's volume. Thus, the molecules composing viruses, bacteria and micromycetes obtain sufficient amount of energy for important bonds that underlie their stable structure to be damaged.

As a result of the natural convection, in a certain period of time all the air contained in the room passes through the volume of the convector and this leads to reduction of the viruses, bacteria and micromycetes in it.

When the user desired temperature is reached the electric heating element 2 in the convector is switched off. After it has been switched off the LED module continues to operate for 10 munities because during the cooling of the electric heating element 2 it still causes natural convection and the LEDs 3 continue to irradiate the passing airflow.

### Examples

In an embodiment shown in Figure 2, the convector, in accordance with the invention, has rated power of 1000W and the following dimensions of the housing (H × W × L): 0.45 × 0.61 × 0.08 m. The electric heating element 2 is of the resistive conductor type and it is installed at 45 mm distance from the bottom of the housing 1, where a large number of air inlet openings 4 are located. The device is intended for heating premises with surface area of 8-12 m² and a volume of 20-30 m³. Above the openings 4, on a load-bearing bus, 5 evenly distributed UVA light emitting diodes 3 are installed. These light emitting diodes 3 are of high power, up to 3W, and 135° emission angle. The selection of powerful diodes with wide emission angle may allow for reduction in their number and at the same time the condition for even illumination of the surface of the electric heating element 2 is fulfilled along with the condition for luminous intensity carrying sufficient amount of energy, whereby together with the joint action of the emitted infrared light from the electric heating element 2 important bonds underlying the stable structure of bacteria, viruses and micromycetes will be damaged. The distance between the electric heating element 2 and the light emitting diodes (LEDs) 3 is 40 mm.

In another embodiment shown on Figure 3, the convector has the same dimensions and characteristics as in the above example. Above the air inlet openings 4, on a load-bearing bus, 20 evenly distributed UVA light emitting diodes 3 are installed. The diodes are of medium or low power (0.2 W - 1W) and emission angle of 135° or in the range of 30-120°. The total radiated power from all the 20 diodes in the UVA spectrum fulfil the condition on luminous intensity carrying sufficient amount of energy whereby together with the joint action of the radiated infrared light from the electric heating element 2 important bonds underlying the stable structure of bacteria, viruses and micromycetes can be damaged. The bigger number of LEDs 3 allows for achieving even illumination of the electric heating element 2 at various angles of radiation (e.g., in the range of 30-120°). The distance between the electric heating element 2 and the light emitting diodes (LEDs) 3 is 40 mm.

Particular variant of the embodiment with external mounting of the sources of UVA is shown on Fig. 5. In this variant the sources of UVA radiation (UV light emitting diodes 3) are located in a separate housing 13, for example aluminium profile open on top. The separate housing 13 is mounted by at least one connecting element 9 to the convector housing 1 underneath the openings 4. The external UVA device may have a separate cable 11 connected to the separate housing 13 for connection to a power source via a control box 12 with an electric plug. The control box 12 manages the mode of the UV light emitting diodes 3. This embodiment allows easy modification of existing convectors by simple installation of the separate housing 13 without the need to change the construction of the convector.

### Studies

There are various approaches and methods for assessing the microbiological pollution in closed spaces and currently among the most efficient ones is the counting of the so-called colony forming units (CFU) on standard Petri dishes, containing nutrient medium, exposed to air for certain time with the aim to provide enough time for biological particles to "sediment" on the surface and then they are placed in an incubator. After incubation at 36°C colonies are formed, and their number is proportionate to the rate of microbiological pollution of the tested air.

Two types of nutrient media have been studied: soybean casein agar (tryptic-soy agar - TSA) and sabouraud dextrose agar with added chloramphenicol (sabouraud chloramphenicol agar - SCA), respectively for evaluation of the bacteria and micromycetes (mucors, moulds, etc.). The results are presented in the following units: CFU/m²/h. The reduction of the number of microorganisms as a result of the action of the presented prototype has been assessed by making and evaluation of the microbial pollution index before and after its operation in a given room for a certain period of time. For the trials the convector which has been used is with rated power of 1000W and the following dimension (H × W × L): 0.45 × 0.61 × 0.08 m, with 5 evenly distributed UVA LEDs installed in the lower part. The distance between the electric heating element 2 and the LEDs 3 is 40 mm. The room where the trials have been carried out has a surface area of 11.8m² and volume of 33m³. The results which have been obtain after 6 hours of operation are shown in table 1.

**Table 1**

| **Type of organisms** | **Medium type** | **Sample** | **CFU, number/1 h/1 m²** | **Reduction of the number of CFU, %** |
|---|---|---|---|---|
| Bacteria | Soybean casein agar (TSA) | control | 1572.7±567.0 | 87 |
| | | sample | 209.7±181.6 | |
| Micromycetes | Sabouraud dextrose agar + chloramphenicol (SCA) | control | 576.7±240.2 | 100 |
| | | sample | 0.0±0.0 | |

Results show significant reduction of the CFU when the convector is used in accordance with the invention, and the reduction of the micromycetes is 100%, i.e., completely eliminated.

For the specialists in this technical field, it will be clear that there are various possible embodiments of the convector which still fall within the scope of the useful model, defined in the enclosed claims.

The reference numbers of the technical properties are included in the claims solely with the purpose of better understanding the claims, and therefore they have no limiting effect with regards to the interpretation of the elements designated by those reference numbers.

## Claims

1. Electric convector consisting of a housing (1) with at least one air inlet opening (4), located in the lower part of the housing (1), and at least one heated air outlet opening (5) located in the upper part of the housing (1), whereby in the housing (1) there is an electric heating element (2) with heating temperature in the range of 200 °C - 450 °C, an electronic control unit adapted to operate the electric heating element (2), and means for connecting to source of electrical energy, whereby the electric convertor further comprises at least one source of UV radiation (3), **characterized in that** the at least one source of UV radiation (3) is a light emitting diode (LED) emitting in UVA spectral range (315-380 nm) with power of more than 150 mW and is located beneath the electric heating element (2), wherein said at least one source of UV radiation (3) is mounted inside the convector housing (1) or outside the convector housing (1) on the path of the incoming airflow.

2. Electric convector in accordance with claim 1, **characterized in that** the UVA source is mounted inside the convector housing (1) between the electric heating element (2) and the inlet opening/s (4), on the path of the incoming airflow.

3. Electric convector in accordance with claim 1, **characterized in that** the UVA source is mounted outside the convector housing (1) underneath the inlet opening/s (4), on the path of the incoming airflow.

4. Electric convector in accordance with claim 1, **characterized in that** the distance between the UV LEDs (3) and the electric heating element (2) is not less than 30 mm.

5. Electric convector in accordance with claim 1, **characterized in that** the electronic control unit comprises at least a microprocessor, memory, timer/time measuring device, internet module and wireless connection module.

6. Electric convector in accordance with any of the preceding claims, **characterized in that** the UV LEDs (3) are distributed evenly above or below the air inlet openings (4).

## Patentansprüche

1. Elektrokonvektor bestehend aus einem Gehäuse (1) mit mindestens einer im unteren Teil des Gehäuses (1) befindlichen Lufteintrittsöffnung (4) und mindestens einer im oberen Teil des Gehäuses (1) befindlichen beheizten Luftaustrittsöffnung (5), wobei es im Gehäuse (1) ein elektrisches Heizelement (2) mit einer Heiztemperatur im Bereich von 200 °C - 450 °C, eine elektronische Steuereinheit zum Betrieb des elektrischen Heizelements ( 2) und Mittel zum Anschließen an eine elektrische Energiequelle verfügbar ist, wobei der Elektrokonvektor ferner mindestens eine UV-Strahlungsquelle (3) umfasst, **dadurch gekennzeichnet, dass** die mindestens eine UV-Strahlungsquelle (3) eine lichtemittierende Diode ( LED), die im UVA-Spektralbereich (315-380 nm) mit einer Leistung von mehr als 150 mW emittiert und unterhalb des elektrischen Heizelements (2) angeordnet ist, wobei die mindestens eine UV-Strahlungsquelle (3) innerhalb des Konvektorgehäuses (1) oder außerhalb des Konvektorgehäuses (1) auf dem Weg des einströmenden Luftstroms angebracht ist.

2. Elektrokonvektor nach Anspruch 1, **dadurch gekennzeichnet, dass** die UVA-Quelle innerhalb des Konvektorgehäuses (1) zwischen dem Elektroheizelement (2) und der/den Eintrittsöffnung(en) (4) auf dem Weg des einströmenden Luftstroms angebracht ist.

3. Elektrokonvektor nach Anspruch 1, **dadurch gekennzeichnet, dass** die UVA-Quelle außerhalb des Konvektorgehäuses (1) unterhalb der/den Eintrittsöffnung(en) (4) auf dem Weg des einströmenden Luftstroms angebracht ist.

4. Elektrokonvektor nach Anspruch 1, **dadurch gekennzeichnet, dass** der Abstand zwischen den UV-LEDs (3) und dem elektrischen Heizelement (2) mindestens 30 mm beträgt.

5. Elektrokonvektor nach Anspruch 1, **dadurch gekennzeichnet, dass** die elektronische Steuereinheit mindestens einen Mikroprozessor, Speicher, Timer/Zeitmesseinrichtung, Internetmodul und Funkverbindungsmodul umfasst.

6. Elektrokonvektor nach einem der vorhergehenden Ansprüche, **dadurch gekennzeichnet, dass** die UV-LEDs (3) gleichmäßig über oder unter den Lufteintrittsöffnungen (4) verteilt sind.

## Revendications

1. Convecteur électrique comportant un boîtier (1) avec au moins une ouverture d'entrée d'air (4), située dans une partie inférieure du boîtier (1), et au moins une ouverture de sortie d'air chaud (5) située dans une partie supérieure du boîtier (1), dans lequel boîtier (1) il y a un élément chauffant électrique (2) avec une température de chauffage selon une plage de 200 °C - 450 °C, une unité de contrôle électronique adaptée pour activer l'élément chauffant électrique (2), et un moyen de connexion à une source d'énergie électrique, dans lequel le convecteur électrique comprend en outre au moins une source de rayonnement UV (3), **caractérisé en ce que** ladite au moins une source de rayonnement UV (3) est une diode d'émission lumineuse (LED) émettant selon une plage spectrale d'UVA de 315-380 nm avec une puissance de plus de 150 mW et est localisée sous l'élément chauffant électrique (2), dans lequel ladite au moins une source de rayonnement UV (3) est montée à l'intérieur du boîtier (1) du convecteur ou à l'extérieur du boîtier (1) du convecteur sur le chemin d'entrée du flux d'air.

2. Convecteur électrique selon la revendication 1, **caractérisé en ce que** la source UVA est montée à l'intérieur du boîtier (1) du convecteur entre l'élément de chauffage électrique (2) et l'ouverture(s) d'entrée d'air (4), sur le chemin d'entrée du flux d'air.

3. Convecteur électrique selon la revendication 1, **caractérisé en ce que** la source UVA est montée à l'extérieur du boîtier (1) du convecteur au dessous de l'ouverture(s) d'entrée d'air (4), sur le chemin d'entrée du flux d'air.

4. Convecteur électrique selon la revendication 1, **caractérisé en ce que** la distance entre les LEDs UV (3) et l'élément de chauffage électrique (2) n'est pas inférieure à 30 mm.

5. Convecteur électrique selon la revendication 1, **caractérisé en ce que** l'unité de contrôle électronique comprend au moins un microprocesseur, une mémoire, un dispositif de mesure temporelle, un module internet et un module de connexion sans fil.

6. Convecteur électrique selon l'une quelconque des revendications précédentes, **caractérisé en ce que** les LEDs UV (3) sont réparties uniformément au-dessus ou au-dessous des ouvertures d'entrée d'air (4).
